(19) Europäisches Patentamt
European Patent Office
Office européen des brevets

(11) **EP 3 304 384 B1**

(12) **EUROPEAN PATENT SPECIFICATION**

(45) Date of publication and mention
of the grant of the patent:
**29.04.2020 Bulletin 2020/18**

(21) Application number: **16727972.8**

(22) Date of filing: **24.05.2016**

(51) Int Cl.:
***G16B 40/00*** (2019.01)

(86) International application number:
**PCT/EP2016/061727**

(87) International publication number:
**WO 2016/193075 (08.12.2016 Gazette 2016/49)**

(54) **METHODS, SYSTEMS AND APPARATUS FOR SUBPOPULATION DETECTION FROM BIOLOGICAL DATA**

VERFAHREN, SYSTEME UND VORRICHTUNG ZUR ERKENNUNG VON SUBPOPULATION AUS BIOLOGISCHEN DATEN

PROCÉDÉS, SYSTÈMES ET APPAREILS DE DÉTECTION DE SOUS-POPULATIONS À PARTIR DE DONNÉES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2015 US 201562169902 P**

(43) Date of publication of application:
**11.04.2018 Bulletin 2018/15**

(73) Proprietor: **Koninklijke Philips N.V.**
**5656 AG Eindhoven (NL)**

(72) Inventors:
• **VOLYANSKYY, Kostyantyn**
**5656 AE Eindhoven (NL)**
• **DIMITROVA, Nevenka**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(56) References cited:
WO-A2-01/73602          US-A1- 2003 097 227
US-A1- 2009 129 647

**EP 3 304 384 B1**

## Description

## Technical Field

The present invention is directed generally to bioinformatics technology. More particularly, various inventive methods, systems and apparatus disclosed herein relate to detection of subpopulations based on biological data.

## Background

Bioinformatics technology provides an efficient means for analyzing biological organisms and is an important aspect of several biological fields. In particular, bioinformatics technological processes have led to significant advancements in genomics and the study and treatment of diseases, including cancer. Cancer, as well as other genome diseases, are characterized by heterogenic patterns of genomic structural variations and gene expression underpinning the evolution from normal to tumor cells. For purposes of clinical studies and, particularly, identification of driver and passenger events in tumor development and proliferation, the ability to interpret and characterize distinctive patterns from available genomic data gains high importance. One method for inferring clonal population structures in cancer employs Bayesian hypothesis testing. This method applies a clustering process that groups sets of sequenced somatic mutations into clonal clusters.

US2003/097227 discloses a method for classifying biological elements into functional families. US2009/129647 relates to methods for identifying patterns in DNA sequence data through spectral analysis. WO01/73602 relates to processing large amounts of biological data by cluster analysis.

## Summary

The complexity and volume of genetic profiles renders it very difficult to efficiently and accurately analyze them for purposes of detecting various subpopulations including, for example, clonal populations reflecting a tumor cell lineage and evolution, and populations of abnormal, normal and disease-specific cell lines. The present disclosure is directed to inventive methods, systems and apparatus for detecting subpopulations of constituents of at least one biological organism. To improve the efficiency of detection of the subpopulations while maintaining a high degree of accuracy, in accordance with an aspect of the present invention, biological data is formulated as discrete-time real-valued vector signals and evaluated using one or more frequency domain analysis procedures. Here, the signals can be defined by the characteristics of a genome, where regions of the genome along the genome length can be denoted as time values. Further, spectral properties of the signals can be obtained through frequency domain analysis procedure(s) and used as features for purposes of distinguishing the subpopulations. Thus, by formulating the biological data as discrete-time real-valued signals and analyzing the signals, the subpopulations can be detected in a highly efficient and accurate manner.

Moreover, in accordance with exemplary aspects, a dissimilarity index can be formed to determine the subpopulations. Here, parent-child pairs of a population tree composed of cohort members can be identified and their similarities can be assessed to construct the dissimilarity index. The dissimilarity index can provide significant advantages, as it can convey subpopulations through highly detectable sharp differences between parent-child pairs, enabling systems, methods and apparatus to accurately detect subpopulations.

Generally, in one aspect, an exemplary system is configured to detect subpopulations of constituents of at least one biological organism. Here, the system is implemented by at least one hardware processor and includes a vector signal formulation module, a frequency domain analyzer and a subpopulation detection module. The vector signal formulation module is configured to formulate biological data compiled from a cohort of the constituents as a set of discrete-time real valued vector signals within at least one data structure of a storage medium. Moreover, the frequency domain analyzer is configured to perform frequency domain analysis on the vector signals of the biological data to compile spectral properties of the vector signals and to associate the spectral properties with the constituents of the cohort. In addition, the subpopulation detection module is configured to identify the subpopulations of the one or more biological organisms by applying a similarity metric to the spectral properties. The subpopulation detection module is further configured to direct the display of a representation of the identified subpopulations.

Similarly, in another aspect, an exemplary method is directed to detecting subpopulations of constituents of at least one biological organism. The method can be implemented by at least one hardware processor. In accordance with the method, biological data compiled from a cohort of the constituents is formulated as a set of discrete-time real valued vector signals within at least one data structure of a storage medium. Further, frequency domain analysis is performed on the vector signals of the biological data to compile spectral properties of the vector signals. Moreover, the spectral properties are associated with the constituents of the cohort. In addition, the subpopulations of the constituents of the one or more biological organisms are identified by applying a similarity metric to the spectral properties.

According to exemplary embodiments, the biological data includes at least one of genomic data or proteomic data. System, method and apparatus embodiments are especially advantageous when applied to genomic or proteomic data due to the complexity and size of the data. As indicated above, embodiments can substantially enhance the efficiency of identifying subpopulations from the data while maintaining a high degree of accuracy.

In one version of exemplary embodiments, the spectral properties include at least one of power spectral density or total spectral energy. The power spectral density and the total spectral energy provide an excellent means for quantifying variances in the biological data, which can be employed to accurately detect distinct properties and differences between subpopulations.

Further, in a version of exemplary embodiments, the biological data includes genomic data and the formulating further comprises formulating regions of a genome of the genomic data as time values. Interpreting regions of a genome as time values is a radically different approach to analyzing genomic data. Further, formulating regions of the genome as time values is an effective way of configuring the data so that frequency domain analysis techniques can be employed to accurately and efficiently identify subpopulations and the degree of dissimilarity between the subpopulations.

In accordance with one optional feature, at least a portion of the genomic data is formulated as at least one linear combination of distinct genomic events. Here, formulating the genomic events as a linear combination provides an efficient means for analyzing the variance of each particular event. For example, the events can include at least one of copy number alteration events, mutations, gene expression data events or methylation data events. Optionally, the frequency domain analysis can include determining at least one of power spectral density or total spectral energy for each genomic event of the distinct genomic events. The analysis of the variance of particular genomic events can be useful in assessing a particular pattern of clonal evolution as well as identifying the aggressiveness and type of disease from which a patient may be suffering.

The identification of subpopulations includes constructing a population tree composed of parent-child pairs of the constituents and forming a population dissimilarity index denoting dissimilarities between a parent and a child of each of the pairs based on the similarity metric. As indicated above, forming a dissimilarity matrix in this way can convey subpopulations through easily detectable sharp differences between parent-child pairs. For example, the identification of subpopulations can include determining a total number of the subpopulations by detecting a total number of distinct peaks of the population dissimilarity index.

In accordance with one optional feature, the formulating of the biological data includes performing a principal component analysis on the data to obtain principal components. In accordance with one exemplary feature, the vector signals on which frequency domain analysis is performed can be composed of the principal components. The principal component analysis can significantly reduce the amount of data analyzed to determine subpopulations, and can thereby enhance the efficiency of method, system and apparatus embodiments. In cases in which the biological data includes genomic data, the principal components can denote linear combinations of genome regions, which in turn identifies the combinations of regions of the genome that exhibit the most significant differences between subpopulations.

In another optional feature, the identification of the subpopulations comprises performing a clustering procedure on a combination of the vector signals and the spectral properties. Here, the total number of distinct peaks can be employed as a height cutoff for the procedure corresponding to the total number of the subpopulations. Thus, the clustering procedure can be guided by the distinct differences conveyed by the dissimilarity index, thereby providing an accurate and efficient means for detecting the subpopulations.

In accordance with exemplary embodiments, a representation of the identified subpopulations can be displayed. For example, the representation can include identified subpopulations as well as descriptive characteristics of intra-subpopulation similarities and inter-subpopulation dissimilarities.

Further, in one aspect, a computer-readable medium comprises a computer-readable program that, when executed on a computer, enables the computer to perform any one or more of the methods described herein. For example, the computer-readable program can be configured to detect subpopulations of constituents of at least one biological organism such that, when the program is executed on a computer, the program causes the computer to perform the steps of any one or more of the method embodiments described herein. The computer-readable medium can be a computer-readable storage medium or a computer-readable signal medium. Alternatively or additionally, the computer readable medium can include an update or other portion of the computer-readable program.

As used herein for purposes of the present disclosure, the term "constituents of at least one biological organism" should be understood to include, but is not limited to, cells, cell lines, bacterial cultures, other microorganisms or patients.

The term biological data should be understood to include, but is not limited to, genomic data, including, for example, one or more of mutations, genome-wide copy number alterations, gene and/or noncoding RNA expression data, DNA methylation data, histone modifications, DNA binding data (e.g. ChIPseq), and/or RNA binding data, and/or other types of genomic data, proteomic data, including, for example, protein expression data, phosphoryltation data, ubiquitination data and/or acetylation data of a biological sample, glucose level data, blood pressure data, weight data, body mass index (BMI) data, dietary data, and/or daily calorie intake, in addition to other types of biological data.

The term "controller" is used herein generally to describe various apparatus relating to the operation of computing devices. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein, or employs dedicated harware. A controller may be implemented with or without employing a processor, and also may be implemented as a combination of dedicated hardware to perform some functions and a microprocessor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more computer-readable storage mediums (generically referred to herein as "memory," e.g., volatile and non-volatile computer memory such as RAM, PROM, EPROM, and EEPROM, floppy disks, compact disks, optical disks, magnetic tape, etc.). In some implementations, the storage mediums may be encoded with one or more programs that, when executed on one or more processors and/or controllers, perform at least some of the functions discussed herein. Various storage mediums may be fixed within a processor or controller or may be transportable, such that the one or more programs stored thereon can be loaded into a processor or controller so as to implement various aspects of the present invention discussed herein. The terms "program" or "computer program" are used herein in a generic sense to refer to any type of computer code (e.g., software or microcode) that can be employed to program one or more processors or controllers. In some implementations, computer readable signal mediums may be encoded with one or more programs that, when executed on one or more processors and/or controllers, perform at least some of the functions discussed herein. For example, a signal medium can be an electromagnetic medium, such as a radio frequency medium, and/or an optical medium, through which a data signal is propagated.

The term "addressable" is used herein to refer to a device (e.g., a controller or processor) that is configured to receive information (e.g., data) intended for multiple devices, including itself, and to selectively respond to particular information intended for it. The term "addressable" often is used in connection with a networked environment (or a "network," discussed further below), in which multiple devices are coupled together via some communications medium or media.

In one network implementation, one or more devices coupled to a network may serve as a controller for one or more other devices coupled to the network (e.g., in a master/slave relationship). In another implementation, a networked environment may include one or more dedicated controllers that are configured to control one or more of the devices coupled to the network. Generally, multiple devices coupled to the network each may have access to data that is present on the communications medium or media; however, a given device may be "addressable" in that it is configured to selectively exchange data with (i.e., receive data from and/or transmit data to) the network, based, for example, on one or more particular identifiers (e.g., "addresses") assigned to it.

The term "network" as used herein refers to any interconnection of two or more devices (including controllers or processors) that facilitates the transport of information (e.g. for device control, data storage, data exchange, etc.) between any two or more devices and/or among multiple devices coupled to the network. As should be readily appreciated, various implementations of networks suitable for interconnecting multiple devices may include any of a variety of network topologies and employ any of a variety of communication protocols. Additionally, in various networks according to the present disclosure, any one connection between two devices may represent a dedicated connection between the two systems, or alternatively a non-dedicated connection. In addition to carrying information intended for the two devices, such a non-dedicated connection may carry information not necessarily intended for either of the two devices (e.g., an open network connection). Furthermore, it should be readily appreciated that various networks of devices as discussed herein may employ one or more wireless, wire/cable, and/or fiber optic links to facilitate information transport throughout the network.

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, a mouse, keyboard, keypad, various types of game controllers (e.g., joysticks), track balls, display screens, various types of graphical user interfaces (GUIs), touch screens, microphones and other types of sensors that may receive some form of human-generated stimulus and generate a signal in response thereto.

It should be appreciated that all combinations of the foregoing concepts and additional concepts discussed in greater detail below (provided such concepts are not mutually inconsistent) are contemplated as being part of the subject matter disclosed herein. It should also be appreciated that terminology explicitly employed herein that also may appear in any disclosure referred to herein should be accorded a meaning most consistent with the particular concepts disclosed herein.

## Brief Description of the Drawings

In the drawings, like reference characters generally refer to the same parts throughout the different views. Also, the drawings are not necessarily to scale, emphasis instead generally being placed upon illustrating the principles of the invention.

FIG. 1 is a high-level block/flow diagram of a system for detecting subpopulations of constituents of at least one biological organism in accordance with exemplary embodiments.

FIG. 2 is a high-level block/flow diagram of a method for detecting subpopulations of constituents of at least one biological organism in accordance with exemplary embodiments.

FIG. 3 is a diagram illustrating an example of genomic data employed to detect subpopulations in accordance with exemplary embodiments.

FIG. 4 is a high-level block/flow diagram of a method for formulating biological data as discrete-time valued vector signals in accordance with exemplary embodiments.

FIG. 5 is a high-level block/flow diagram of a method for performing frequency domain analysis on the vector signals of the biological data to obtain spectral properties of the signals in accordance with exemplary embodiments.

FIG. 6. is a high-level block/flow diagram of a method for identifying subpopulations by applying a similarity metric to the spectral properties in accordance with exemplary embodiments.

FIG. 7 is a diagram illustrating an example of a population dissimilarity index in accordance with exemplary embodiments.

FIG. 8 is a diagram illustrating an example of a representation of subpopulations that can be formed and displayed in accordance with exemplary embodiments.

FIG. 9 is a high-level block/flow diagram of an exemplary computer system that can implement one or more exemplary embodiments.

## Detailed Description

Bioinformatic analysis of genomic data is generally very difficult due to the complexity and size of the data. The analysis is particularly difficult when it is applied to a very large cohort of patients, cell lines and/or cells for purposes of detecting subpopulations, which can include, for example, clonal populations of disease cells or different cell-lines associated with a disease. To improve the accuracy and efficiency of detecting subpopulations, Applicants have recognized and appreciated that it would be beneficial to formulate biological data as one or more discrete-time real-valued vector signals. For example, the signals can be defined by characteristics of a genome, where the regions of the genome along its length can be designated as time values. Accordingly, using one or more frequency domain analysis procedures, the signals can be evaluated to obtain spectral properties that can be employed as feature vectors to distinguish the subpopulations. Furthermore, to determine the subpopulations, a dissimilarity index is formed based on sequential identification of parent-child pairs of a population tree composed of cohort members. The dissimilarity index conveys subpopulations through highly detectable and visible sharp differences between parent-child pairs, thereby providing an efficient and elegant means for identifying the subpopulations.

The identification of subpopulations as described herein can be employed as a diagnostic tool. For example, the identification of subpopulations can be employed in clinical applications for purposes of discerning patterns of clonal evolution and tumor heterogeneity in assessments of aggressiveness of the tumor sample. In particular, when applied to detect clonal cell populations, high dissimilarity indices between the subpopulations indicate high heterogeneity with multiple clonal and sub-clonal populations. This insight provides significant advantages in the treatment of cancer, as well as other diseases. Thus, embodiments can be employed to aid in the treatment of diseases. For example, the embodiments can be utilized in therapy design. Here, the identification of subpopulations is particularly advantageous, as doctors can tailor drugs and inhibitors to each subpopulation, rather than using one inhibitor on an average target. Thus, in this way, certain subpopulations that are shown by embodiments to be particularly aggressive can be specifically targeted to treat a patient. Embodiments described herein can also be used to discover new population outgrowth in bacterial infections and can be used to distinguish between hospital acquired infections and community acquired infections.

In view of the foregoing, various embodiments and implementations of the present invention are directed to methods, systems and apparatus for detecting subpopulations of constituents of at least one biological organism. The embodiments can be employed to, for example, classify genomic and/or transcriptomic events, characterize clonal cell populations, and extract valuable clinical information, such as tumor progression patterns, prognosis of treatment plan efficacy, and patient risk. Further, embodiments can include a pattern recognition tool that can detect clonal populations based on genomic data including, for example, mutations, genome-wide copy number alterations, gene and/or noncoding RNA expression data, DNA methylation data, histone modifications, DNA binding data (e.g. ChIPseq), and/or RNA binding data, in addition to other types of genomic data. Alternatively or additionally, clonal populations can be detected from proteomic data, which can be extracted from mass spectrometry methods and can be incorporated into the integrated analysis. Mertins et al., "Integrated proteomic analysis of post-translational modifications by serial enrichment," Nature Methods 10, 634-637 (2013) describes an example of a mass spectrometry method. The proteomic data can include protein expression data, phosphoryltation data, ubiquitination data and acetylation data of a biological sample. Moreover, exemplary embodiments can build phylogenetic trees to depict the results from this analysis. As discussed herein below, exemplary embodiments can apply a combination of data quantization, principal component analysis, spectral frequency methods, phylogenetic paradigm, and clustering methodology to identify and characterize clonal evolution. Thus, various analytic tools can be combined under one umbrella to maximize performance in population detection. In accordance with exemplary embodiments, intra- and inter-cell heterogeneity can be characterized in automated fashion for purposes of genome disease studies and patient clinical assessment. In addition, the embodiments can also detect subpopulations in bacterial evolution for infectious disease management.

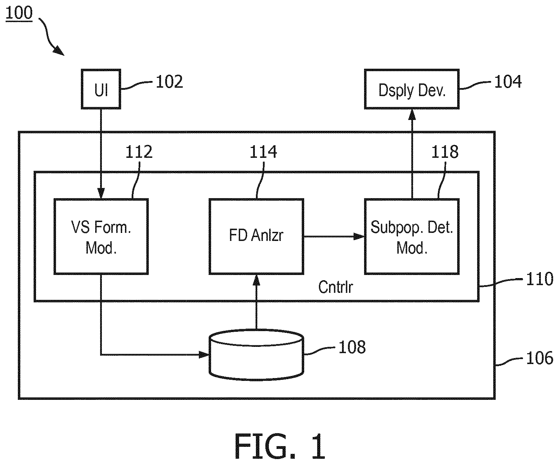

With reference to FIG. 1, an exemplary system 100 system for detecting subpopulations of constituents of at least one biological organism in accordance with exemplary embodiments is illustratively depicted. The system 100 can include a vector signal formulation module (VS Form. Mod.) 112, a frequency domain analyzer (FD Anlzr) 114 and a subpopulation detection module (Subpop. Det. Mod.) 118. Each of the system components 112, 114 and 118 can be implemented by a controller (Cntrlr) 110, which can be one or more hardware processors that are part of a hardware computing system 106. The computing system 106 can also include a storage medium 108, and the system 100 can include a user-interface (UI) 102 and a display device (Dsply Dev.) 104.

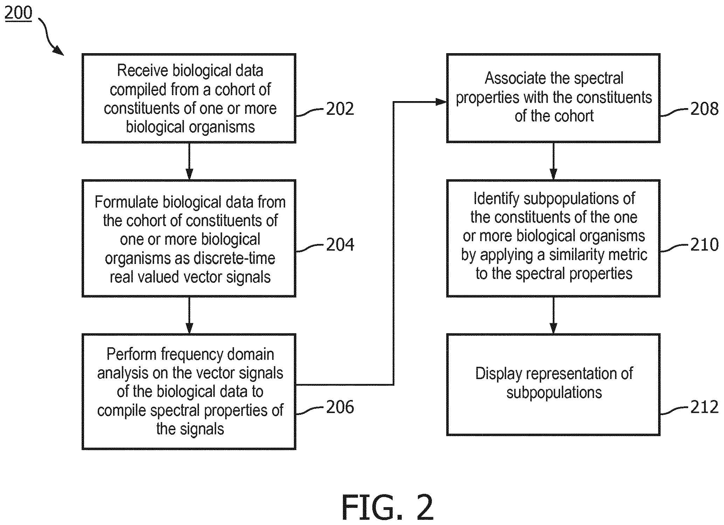

Referring to FIG. 2, with continuing reference to FIG. 1, an exemplary method 200 for detecting subpopulations of constituents of at least one biological organism is illustratively depicted. Here, the constituents can be cells, e.g., clonal cells, or cell lines of one or more organisms. Alternatively or additionally, the constituents can be the biological organisms themselves, including, for example, patients or even bacterial cultures. The method 200 can be applied to detect subpopulations of any one or more of these constituents based on biological data, including, for example, genomic data and/or proteomic data, compiled from the constituents. It should be noted that the method 200 can be performed by the system 100 or 106. For example, the steps of the method 200 can be instructions of a program that can be stored on the storage medium 108 and executed by the controller 110, as, for example, discussed herein below.

The method 200 can begin at step 202, at which the system 106 can receive biological data compiled from a cohort of constituents of one or more biological organisms through the user-interface 102. The biological data can include at least one of genomic data or proteomic data. For each member of the cohort, the genomic data can include, as discussed above, one or more of genome-wide copy number alterations, gene expression data, methylation data, and/or other types of genomic data. Alternatively or additionally, as noted above, proteomic data can include protein expression data, phosphoryltation data, ubiquitination data and acetylation data of a biological sample. Proteomic data is the functional readout of the genomic architecture and many gene biological processes. The genomic and/or proteomic data may be composed of one of the types of data described above or any combination of the different types of data. As discussed herein below, the copy number alterations can denote deletions and amplifications for various regions of a genome for each member of the cohort. Gene expression data and methylation data represent additional types of genome characterization in terms of over/under expression of genes and degree of gene silencing or activation in a given biological organism. These data are provided as quantitative variables derived from measurement procedures and can be part of the input received at step 202. It should also be noted that although genomic and proteomic data are described here as examples, the biological data can additionally or alternatively include any type of data that characterizes populations. For example, the biological data can include measurements on diabetic patients, which can, in turn, include glucose level data, blood pressure data, weight data, body mass index (BMI) data, dietary data, and/or daily calorie intake. As understood by those skilled in the art based on the present Specification, the data can be formulated and analyzed in a manner similar to the examples described herein below with respect to genomic data. The method 200 can employ the data to determine subpopulations with certain clinical characteristics, including populations that have additional small vasculature complications.

At step 204, the vector signal formulation module 112 can formulate the biological data compiled from the cohort of the constituents of the biological organism(s) as a set of discrete-time real valued vector signals within at least one data structure of the storage medium 108. For example, genomic data compiled from the cohort can be formulated

as follows:

$$
\begin{array}{ccccccccc}
CNA_{1,1} & \dots & CNA_{M,1} & GE_{1,1} & \dots & GE_{M,1} & M_{1,1} & \dots & M_{M,1} \\
CNA_{1,2} & \dots & CNA_{M,2} & GE_{1,2} & \dots & GE_{M,2} & M_{1,2} & \dots & M_{M,2} \\
CNA_{1,3} & \dots & CNA_{M,3} & GE_{1,3} & \dots & GE_{M,3} & M_{1,3} & \dots & M_{M,3} \\
\cdot & \cdot & \cdot & \cdot & \cdot & \cdot & \cdot & \cdot & \cdot \\
\cdot & \cdot & \cdot & \cdot & \cdot & \cdot & \cdot & \cdot & \cdot \\
\cdot & \cdot & \cdot & \cdot & \cdot & \cdot & \cdot & \cdot & \cdot \\
CNA_{1,N} & \dots & CNA_{M,N} & GE_{1,N} & \dots & GE_{M,N} & M_{1,N} & \dots & M_{M,N}
\end{array}
\qquad (1)
$$

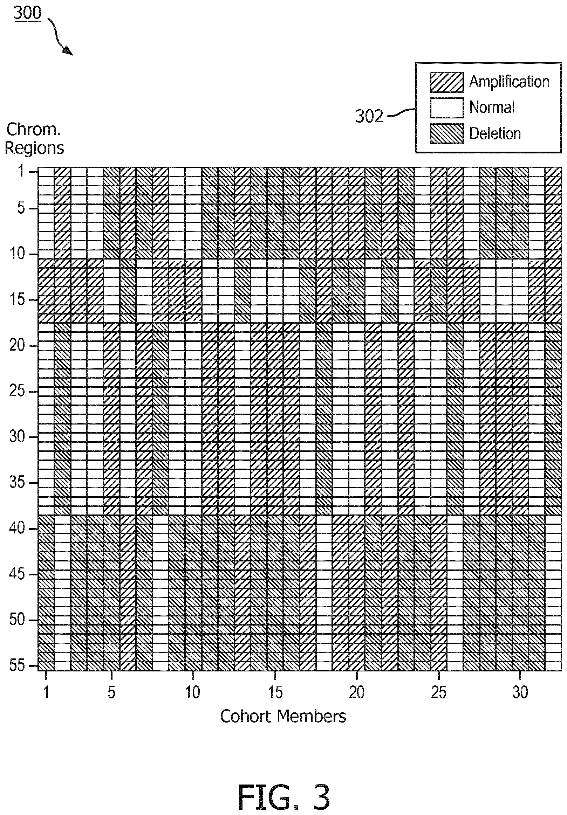

In this particular example, the genomic data consists of genome-wide copy number alteration (CNA), gene expression data (GE), methylation data (M). However, it should be understood that the matrix can be composed of one of these types of data or any sub-combination of these types of data or other types of data discussed above. Further, each set of columns denotes a particular member of a cohort, which can be, for example, a particular cell. For example, if the cohort members are cells, the cells are denoted by the first subscript in the elements of matrix (1), where $CNA_{1,m}$, $GE_{1,m}$ and $M_{i,m}$ denote copy number alteration data, genome expression data and methylation data of cell 1, $CNA_{2,m}$, $GE_{2,m}$ and $M_{2,m}$ denote copy number alteration data, genome expression data and methylation data of cell 2, etc.. Here, m denotes an arbitrary chromosome region of a genome, where the genome of each cell in the cohort is delineated by 1, 2, 3 ... N regions along the genome length. The delineated regions are denoted by the rows in Matrix (1). For example, $CNA_{1,1}$, $GE_{1,1}$ and $M_{1,1}$ denote copy number alteration data, genome expression data and methylation data, respectively, of region 1 of cell 1, $CNA_{1,2}$, $GE_{1,2}$ and $M_{1,2}$ denote copy number alteration data, genome expression data and methylation data, respectively, of region 2 of cell 1, $CNA_{2,2}$, $GE_{2,2}$ and $M_{2,2}$ denote copy number alteration data, genome expression data and methylation data, respectively, of region 2 of cell 2, etc. Thus, $CNA_{n,m}$ can denote a normal alternation, a deletion or an amplification in region m of the genome of cell n, while $GE_{n,m}$ can denote values of genes that are expressed at region m of the genome of cell n. FIG. 3 illustrates a graph 300 of an example of the copy number alteration data for a cohort of 32 cells for 55 chromosome (Chrom.) regions of the genome, where the deletions, amplification, and normal characteristics are designated in the legend 302. The delineated regions of the genome can also be received at step 202 and subsequently arranged as a column vector, which can be stored in a storage structure within the storage medium 108 and can be a reference employed by the controller 110 to map elements of matrix (1) to particular genome regions. In matrix (1), each column is formulated as a discrete-time real valued signal, where a column element denotes a value of the signal and each region 1, 2, 3 ... N is denoted as time values or time intervals. As discussed herein below, formulating the biological data in this way can substantially improve the efficiency and accuracy of detecting subpopulations within the cohort. Quantization of the continuous signals can be performed by employing histograms and appropriate percentiles, as necessary. For example, copy number alterations may be represented as rounded integers referred to as copy number states, with '0' representing region deletion, '1' representing a single copy of a genome region, '2' two copies of a genome region, and so on. For gene expression or methylation, thresholds based on percentiles may be applied to distinguish between over- and under- expression of genes as well as silenced versus active regions for a given cohort.

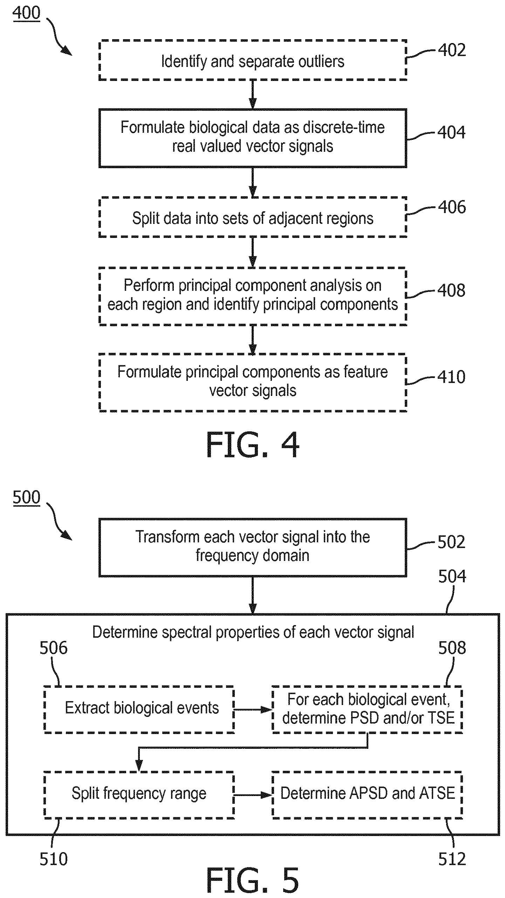

In accordance with one embodiment, the matrix (1) can be the vector signals analyzed in step 206 and subsequent steps. Alternatively, the matrix (1) can be further processed to obtain vector signals that are analyzed in step 206 and subsequent steps. For example, with reference to FIG. 4 with continuing reference to FIGS. 1 and 2, a method 400 for formulating biological data as discrete-time valued vector signals is illustratively depicted. The method 400 can be performed to implement step 204 of the method 200. The method 400 can optionally begin at step 402, at which the vector signal formulation module 112 can identify outliers in the biological data received at step 202 and can separate the outliers from the biological data. For example, the signal formulation module 112 can apply one or more of a variety of approaches, including at least one of a Mahalanobis distance method, a principal component analysis (PCA) method or a frequency based method. Here, the signal formulation module 112 can apply one of these methods, all of the methods or any sub-combination of the methods to identify and separate outliers. For each of these methods, the biological data received at step 202 can be composed in a data matrix.

In the Mahalanobis distance method, the signal formulation module 112 can split the data matrix, which would typically have a high dimension, into regions. Here, each data category can be grouped in the matrix, as, for example, adjacent columns. For example, genome-wide copy number alteration data can be grouped in a set of adjacent columns, gene expression data can be grouped in a set of adjacent columns, methylation data can be grouped in a set of adjacent columns, etc. The signal formulation module 112 splits the matrix such that each category set is split into multiple regions, so that any given region is composed of data from only one category. For each region and data category, signal formulation module 112 can compute a mean value estimate $M(X)$ and a covariance estimate $C(X)$ as follows:

$$M(X) = Average(X) \qquad (2)$$

$$C(X) = \frac{1}{n-1} \sum (x - M(X))^T (x - M(X)) \qquad (3)$$

where $X$ denotes a data category, which can be, for example, a copy number alteration category, a gene expression data category or a methylation data category, $x$ denotes a value or element in the region, and $n$ here denotes the number of elements in the region. The signal formulation module 112 can compute the Mahalanobis distance $MD(x,X)$ for each element $x$ in quadratic form as follows:

$$MD(x, X) = (x - M(X)) C^{-1}(X)(x - M(X)) \qquad (4)$$

Further, the signal formulation module 112 can detect outliers as points with large Mahalanobis distances that are above a threshold. The signal formulation module 112 can also evaluate the Mahalanobis distances using a chi-squared ($\chi^2$) distribution of degrees of freedom identified from the region dimension (n-1).

In the PCA analysis method, the signal formulation module 112 can linearly transform (rotate) the original data matrix such that the correlation matrix is diagonalized in the transformed space. Here, the signal formulation module 112 can split the correlation matrix into regions, as for example discussed above with regard to the Mahalanobis distance method, and can select the number of principal components based on the threshold of variance captured by these components. For example, the threshold can be chosen to be 90%. The signal formulation module 112 can compute the Mahalanobis distance on the obtained principal components as discussed above with respect to equations 2-4 and can apply the chi-squared test to identify abnormally high values as outliers, as discussed above.

In the frequency-based method, the signal formulation module 112 detects outliers by employing power spectrum estimation as points with a high value of power attributed to high frequencies. In this approach, the signal formulation module 112 computes a Discrete Fast Fourier Transform (DFFT) of each sample. Here, a sample can be composed of a category of biological data for a cohort member. For example, the sample can be composed of a column of matrix (1). The signal formulation module 112 can then estimate a power spectrum distribution and can quantize the estimated power spectrum into low, intermediate, and high regions. Further, the signal formulation module 112 can perform clustering of data points on the quantized power spectrum regions and can identify outliers as members of the distinct cluster in the high frequency region.

At step 404 of the method 400, the vector signal formulation module 112 can formulate the biological data as discrete-time real valued vector signals. For example, the signal formulation module 112 can formulate the original data received at step 202 as discussed above with regard to step 204 and matrix (1). Alternatively, the signal formulation module 112 can formulate the processed biological data, in which outliers were removed at step 402, as discussed above with regard to step 204 and matrix (1).

Optionally, at step 406, the vector signal formulation module 112 can split the biological data into sets of adjacent regions. For example, the vector signal formulation module 112 can split genome-wide data, e.g., the matrix (1) formulated in step 404, into sets of adjacent chromosome regions or rows per data category to simplify any subsequent PCA analysis. The size of the sets are controlled such that the number of chromosome regions in the set is less than the total number M of members of the cohort. Further, each set is composed of biological data of one data category. For example, a set can be composed of genome-wide copy number alteration data, gene expression data, methylation data, or another category of data.

Optionally, at step 408, the vector signal formulation module 112 can perform a principal component analysis procedure on each set and can identify the principal components that capture or exceed a preset value of a variance threshold T. For example, T can be set to $T \geq 90\%$. Here, to implement the PCA procedure, the vector signal formulation module 112 can linearly transform (rotate) each set of regions such that the correlation matrix for the corresponding set is diagonalized in the transformed space. Further, the vector signal formulation module 112 can identify independent axes in which the data distribution has the highest variance, *e.g.,* above the threshold T, and thereby identify linear combinations of chromosome/genome regions as the principal components in cases in which the biological data includes genomic data. The PCA analysis can significantly reduce the number of chromosome regions under consideration.

Optionally, at step 410, the vector signal formulation module 112 can formulate the principal components as feature vector signals. For example, the vector signal formulation module 112 can formulate the principal components determined at step 408 as discrete-time real valued vector signals. For example, for genomic data, regions of the chromosome can be formulated as time values, as discussed above with respect to step 204 and matrix (1). Thus, the feature vector signals can be composed of the principal components. Further, the feature vector signals determined from the sets of regions can be grouped under their respective categories in a matrix. For example, all feature vector signals determined from genome-wide copy number alteration data can be grouped in a feature vector signal matrix, feature vector signals determined from gene expression data can be grouped in the feature vector signal matrix, etc., similar to the groupings illustrated in matrix (1) above.

At step 206 of the method 200, the frequency domain analyzer 114 can perform frequency domain analysis on each of the vector signals of the biological data to compile spectral properties of the vector signals. Here, the frequency domain analyzer 114 can access the vector signal formulations from one or more storage structures of the storage medium 108. Further, the vector signals accessed from the storage medium 108 can be the raw data, such as, for example, the matrix (1) of the genomic data discussed above, the vector signals formulated at step 404, and/or can be the vector signals composed of principal components formulated at step 410. The spectral properties can be at least one of power spectral density or total spectral energy. For example, FIG. 5 illustrates one exemplary method 500 for performing the frequency domain analysis that can be executed to implement step 206 in accordance with exemplary embodiments.

The method 500 can begin at step 502, at which the frequency domain analyzer 114 can transform each of the vector signals into the frequency domain. For example, the frequency domain analyzer 114 can perform a Discrete-Time Fast Fourier Transform (DT FFT) to transform each of the vector signals into the frequency domain. To illustrate how the method 500 can be implemented, an example of analyzing genome-wide copy number data is provided herein below. However, it should be understood that the method can be applied to other types of biological data in a similar manner. In accordance with exemplary aspects, a copy number profile is represented as an array of numbers, where each number describes specific region copy number value. In this example, three major events describing the status of each region is considered: Normal (N), Deletion (D) and Amplification (A). The Deletion event can include sub-categories denoted as Partial Deletion (PD) and Complete Deletion (CD). In addition, the Amplification event can include sub-categories Small Amplification (SA), Moderate amplification (MA) and Large amplification (LA), where these sub-categories can be user-defined. Here, at least a portion of the genomic data can be formulated as at least one linear combination of distinct genomic events. As indicated above, the genomic events can include at least one of copy number alteration events, gene expression data events or methylation data events. In this example, a copy number profile, which is the copy number data for a member (e.g., a cell) of the cohort, is characterized in terms of a combination of (N), (D), and (A) events. Thus, by applying a DF FFT to each of the vector signals of the copy number profile data, the resulting linear combination can be obtained for each vector signal as follows:

$$ CN(t) = \alpha_N X_N(t) + \alpha_{PD} X_{PD}(t) + \alpha_{CD} X_{CD}(t) + \alpha_{SA} X_{SA}(t) + \alpha_{MA} X_{MA}(t) + \alpha_{LA} X_{LA}(t) \quad (5) $$

where $CN(t)$ is the DF FFT of the copy number profile, $t$ is a discrete-time variable describing the chromosome region position in the copy number profile, $\alpha_i \in \{0,1\}$ are Boolean coefficients representing occurrence of a specific event, $i \in \{N, PD, CD, SA, MA, LA\}$, $X_i(t) \in \{0,1\}$ is a discrete-time function describing an occurrence ('1') or absence ('0') of event $i$ at region $t$.

At step 504, the frequency domain analyzer 114 can determine spectral properties of each vector signal. In accordance with an exemplary embodiment, the spectral properties include at least one of power spectral density or total spectral energy. However, other spectral properties may be determined and applied by the method 200/500. According to one exemplary embodiment, the frequency domain analyzer 114 can determine the spectral properties by performing steps 506-512.

At step 506, the frequency domain analyzer 114 can extract biological events from each frequency domain vector signal. For example, the frequency domain analyzer 114 can extract genomic events from each frequency domain vector signal. Continuing with the copy number example described above, the frequency domain analyzer 114 can extract $(\alpha_i, X_i)$, $i \in \{N, PD, CD, SA, MA, LA\}$ from $CN_j$, where $j$ denotes a copy number profile or a cohort member, $j = 1,2,...,M$, where $M$ is the total number of copy number profiles or cohort member, which can be a cell, evaluated here. Each event is defined by the set of threshold parameters $\{i_{min}, i_{max}\}$, and is detected every time $CN_j(t) \in \{i_{min}, i_{max}\}$.

At step 508, the frequency domain analyzer 114 can, for each of the biological events of the corresponding vector signal, determine at least one of a power spectral density (PSD) or a total spectral energy (TSE). In other words, continuing with the copy number example described above, the frequency domain analyzer 114 can obtain the PSD and the TSE for every $X_{ij}$, $i \in \{N, PD, CD, SA, MA, LA\}$, $j = 1,2,...,M$. PSD and TSE computation can be performed using methods of digital signal processing (DSP) and can be based on DT FFT, signal periodogram, Bartlett's method, or Welch's method, for example.

At step 510, the frequency domain analyzer 114 can optionally split the frequency range. For example, with respect to genomic data, because there may be a large number of events in a short range of chromosomal length, splitting the frequency region can provide finer feature characteristics of the vector signals for purposes of identifying subpopulations, thereby improving the accuracy of the subpopulation detection. Here, the frequency domain analyzer 114 can split the frequency range into low, medium or high frequency segments. However, it should be understood that the frequency range can be split into a larger number of segments and/or can be split equally and/or unequally.

At step 512, the frequency domain analyzer 114 can determine the average power spectral density (APSD) and the average total spectral energy (ATSE) for each vector signal in each of the frequency segments. For example, the frequency domain analyzer 114 can determine, for each of the frequency segments, the APSD and the ATSE of each genomic event $X_{ij}$, $i \in \{N, PD, CD, SA, MA, LA\}$, of each member of the cohort $j = 1, 2, ..., M$. Thus, each of the genomic events in the copy number example above can have three values of the APSD in the low, medium and high frequency segments, respectively, and three values of the ATSE in the low, medium and high frequency segments, respectively.

Referring again to FIG. 2, the method 200 can proceed to step 208, at which the frequency domain analyzer 114 can associate the spectral properties with the constituents of the cohort. In particular, the frequency domain analyzer 114 can associate each of the spectral properties with the respective cohort member from which the spectral properties were obtained. For example, the frequency domain analyzer 114 can arrange all of the APSD and ATSE data obtained from a given cohort member $j$ in one or more columns associated with that given cohort member/constituent $j$ in the feature vector matrix. In addition, the frequency domain analyzer 114 can arrange the APSD and ATSE data for each of the other cohort members/constituents in the feature vector matrix in the same manner. Here, the APSD and ATSE data from different data categories, *e.g.,* methylation data, copy number data, and gene expression data, can be grouped together in the matrix or separately, as long as the arrangement is consistent among the cohort members and the spectral property data is associated with the respective cohort members. It should be noted that, in accordance with exemplary aspects, the frequency domain analyzer 114 can validate the spectral property data by simulating copy number profiles using a sequence of low pass, high pass and band pass, notch filters with computed power spectral density characteristics and comparing the clustering distribution of the copy number profiles in the enriched set.

As discussed herein below, the feature vector matrix can be employed to identify subpopulations of constituents of one or more biological organisms. Here, the feature vector matrix can be composed of, for example, the spectral property data alone, the spectral property data combined with the PCA feature vectors in the feature vector signal matrix formulated, for example, at step 410, as additional columns of the feature vector signal matrix, or the spectral property data combined with the preliminary biological data matrix, for example, the genomic data matrix (1) discussed above, with or without outlier removal, as additional columns in the preliminary biological data matrix. In each of these cases, the frequency domain analyzer 114 can construct the feature vector matrix and store it within one or more storage structures of the storage medium 108 and/or provide the feature vector matrix directly to the subpopulation detection module 118 for further analysis.

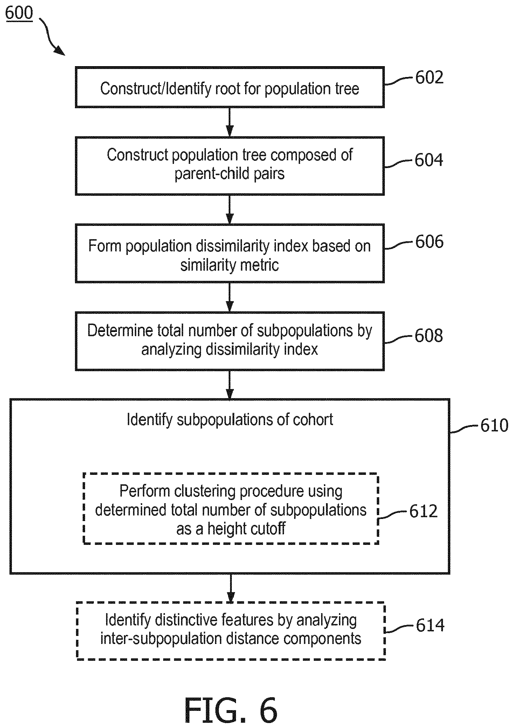

At step 210, the subpopulation detection module 118 can identify the subpopulations of the constituents of the one or more biological organisms by applying a similarity metric to the spectral properties. In accordance with one exemplary embodiment, the subpopulation detection module 118 can identify the subpopulations by performing the method 600 of FIG. 6. The method 600 can begin at step 602, at which the subpopulation detection module 118 can construct or identify a root for a population tree composed of the constituents. In other words, the subpopulation detection module 118 can construct/identify a relevant candidate as a common parent of the cohort members. This candidate can be a cohort member. Alternatively, the subpopulation detection module 118 can quantitatively construct a data point representing, for example, a characteristic parent genomic profile, which can be an average profile or an ideal normal pattern, as the root in the population tree. For example, for the copy number genomic data category, the most normal copy number for the constituents can be identified or selected as the root. In addition, for the gene expression genomic data category for constituents of cells, average values of tissues of normal cells can be employed as the root of the population tree.

At step 604, the subpopulation detection module 118 can construct a population tree composed of parent-child pairs of the constituents of the one or more biological organisms. For example, the subpopulation detection module 118 can construct the population tree by first identifying a cohort member closest to the root, and iteratively assigning un-assigned cohort members to the tree by identifying parent-child pairs using a similarity metric. Here, the portion of the feature vector matrix associated with a given cohort member/constituent can be compared to the corresponding feature vectors of the root, or if the child or children of the root has already been identified, to the portion of the feature vector matrix associated with the parent(s) most recently added to the tree during the iterations. As noted above, the feature vectors associated with the cohort members and included in the feature vector matrix can be composed of, for example, the spectral property data alone, the spectral property data combined with the PCA feature vectors in the feature vector signal matrix formulated, for example, at step 410, as additional columns of the feature vector signal matrix, or the spectral property data combined with the preliminary biological data matrix, for example, the genomic data matrix (1) discussed above, with or without outlier removal, as additional columns in the preliminary biological data matrix. In addition, the subpopulation detection module 118 can receive the feature vector matrix from the frequency domain analyzer 114 or can retrieve the feature vector matrix from the storage medium 108. Further, to determine a parent-child pair, the subpopulation detection module 118 can apply a similarity metric including, for example, one or more distance measures such as a Euclidean distance measure, a Manhattan distance measure, etc.

Thus, at step 604, the subpopulation detection module 118 can apply the distance measure to the feature vectors of each of the cohort members to determine their respective distances from the root. Further, the subpopulation detection module 118 can select, as the child or children of the root, the cohort member or members with the feature vectors having the closest/shortest distance measure to the feature vector of the root. To construct the next level of the population tree, the subpopulation detection module 118 repeats the process. For example, the identified child or children of the root are denoted as the parent or parents in the next level of the population tree. The subpopulation detection module 118 can again apply the distance measure to the feature vectors of each of the remaining, unassigned cohort members to determine their respective distances from the parent under consideration. In addition, the subpopulation detection module 118 can select, as the child or children of the parent under consideration, the cohort member or members with the feature vectors having the closest/shortest distance measure to the feature vector of the parent. The subpopulation detection module 118 can repeat the process until all of the cohort members have been assigned to at least one parent-child pair. As a result, the subpopulation detection module 118 can obtain the population tree, where each cohort member is associated with its parent.

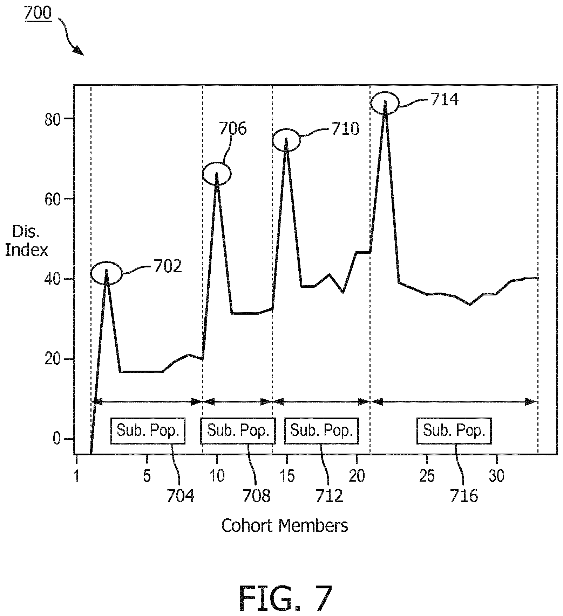

At step 606, the subpopulation detection module 118 can form a population dissimilarity index based on the similarity metric. In particular, the subpopulation detection module 118 can form a dissimilarity index that denotes dissimilarities between a parent and a child of each of the pairs based on the similarity metric. Thus, the population dissimilarity index can be a population tree dissimilarity measure. FIG. 7 is a diagram illustrating an example of a population dissimilarity index (Dis. Index) 700. In FIG. 7, the cohort members are arranged on the horizontal axis, while the dissimilarity index values are provided on the vertical axis. Here, the cohort members are arranged in the order established through construction of the population tree. For example, adjacent cohort members are parent-child pairs, where member 1 is the parent of member 2, member 2 is the parent of member 3, etc. Thus, the dissimilarity index curve denotes the dissimilarity between a parent and a child in the parent-child pairs. In accordance with one exemplary embodiment, the dissimilarity index value can denote the similarity metric discussed above. For example, the dissimilarity index can be the Euclidean distance or the Manhattan distance between a child and a parent in each of the parent-child pairs.

In accordance with another embodiment, the dissimilarity index can be formed based on one or more biological events. For example, the dissimilarity index can be formed based on one or more genomic events. For example, for each parent-child pair, the subpopulation detection module 118 can identify the percentage of genome affected by a specific genomic event (e.g., for the copy number data category, amplification, deletion) and can compute the (weighted) average of genomic event occurrence across all event categories. For example, the subpopulation detection module 118 can assess the genomic event(s) that occur in the child which are not present in the genome of the parent of a given parent-child pair. For example, for copy number data, if the child exhibits PD, SA, etc. events that were not exhibited in the parent, then the subpopulation detection module 118 determines the percentage of the genome that was affected by these new PD events, the percentage of the genome that was affected by the new SA events, etc. Further, the subpopulation detection module 118 can take the average of the percentages for each of the different genomic events and can denote this average as the dissimilarity index. The average can include events from one biological data category or a plurality of biological data categories, including, for example, copy number data, gene expression data, methylation data, etc. Optionally, in accordance with exemplary aspects, the average can be weighted. For example, if certain biological events are considered to be more important with respect to the particular research or purpose for which the method is applied, then these events can be weighted over other events in the average. For this purpose, the subpopulation detection module 118 can link categories of events to quantization levels. For example, in case of copy number alterations, categories could be Minor, Moderate, Large or Abnormal Amplification or Deletion. In the case of gene expression, the categories can be composed of Down-Regulation, Normal, or Over-Expression. The dissimilarity index illustrated in FIG. 7 was determined based on a weighted average of genomic event occurrence. It should be noted that this dissimilarity index is also based on the similarity metric discussed above, as the parent-child pairs evaluated to determine the average of genomic event occurrence were identified based on the similarity metric.

At step 608, the subpopulation detection module 118 can determine a total number of the subpopulations by analyzing the dissimilarity index. For example, the subpopulation detection module 118 can determine the total number of the subpopulations by detecting the number of sharp or substantial differences between children and parents in parent child pairs. For example, when the population dissimilarity index is constructed as illustrated in FIG. 7, the subpopulation detection module 118 can determine the total number of the subpopulations by detecting a total number of distinct peaks of the population dissimilarity index. Here, each peak represents a distinct subpopulation. For example, peak 702 denotes subpopulation (Sub. Pop.) 704, peak 706 denotes subpopulation 708, peak 710 denotes subpopulation 712, and peak 714 denotes subpopulation 716.

At step 610, the subpopulation detection module 118 can identify the subpopulations of the cohort of constituents of the one or more biological organisms and can store the identified subpopulations in one or more storage structures of the storage medium 108. For example, in one exemplary embodiment, the subpopulation detection module 118 can identify the subpopulations as the subpopulations denoted by sharp or substantial differences between children and parents in parent child pairs of the dissimilarity index. For example, the subpopulations can be identified as the sets of cohort members denoted by distinctive peaks of the dissimilarity index. As illustrated in FIG. 7, the subpopulations can be identified as the subpopulations 704, 708, 712 and 716. Here, the cohort member at the peak is the first cohort of a new subpopulation. As noted above, the cohort members along the horizontal axis are ordered as parent-child pairs according to the population tree constructed at step 604. In accordance with other embodiments, the subpopulations can be detected based on a clustering scheme.

For example, to implement step 610, optionally, at step 612, the subpopulation detection module 118 can perform a hierarchical clustering procedure by using the determined total number of subpopulations as a height cutoff. Here, the subpopulation detection module 118 can perform the clustering procedure on the feature vector matrix. As noted above, the feature vector matrix can, for example, be the spectral properties, a combination of the spectral properties and the PCA components, or a combination of the spectral properties and the original biological data, such as matrix (1), with or without outlier removal. In addition, the subpopulation detection module 118 can perform any one of a variety of clustering procedures, including, for example, hierarchical clustering, fuzzy clustering, density based spatial clustering of applications with noise (DBSCAN), k-means clustering, etc. In accordance with one preferred embodiment, the subpopulation detection module 118 can run a hierarchical clustering routine on a feature vector matrix from the data. The matrix can be composed of the real values extracted from experiments/measurements and input at step 202, or can be values obtained after applying a multidimensial reduction method to extract PCA components and/or spectral properties, as noted above. Further, the subpopulation detection module 118 can employ a height cutoff corresponding to the number of subpopulations determined at step 608. Thus, the subpopulation detection module 118 can construct a hierarchical cluster tree and can select the level of the tree corresponding to the determined number of clusters, which would be four clusters in the example illustrated in FIG. 7. Further, the subpopulation detection module 118 can identify the subpopulations as the clusters at the selected height cutoff.

Optionally, at step 614, the subpopulation detection module 118 can identify distinctive features by analyzing inter-subpopulation/cluster distance components. For example, such features can be genome alterations indicated by any of the genomic events discussed above. The identification can be accomplished by characterizing a subgroup representative using, for example, averaging across the subgroup members, and then making comparisons between representatives in terms of their similarities and dissimilarities. Here, the subpopulation detection module 118 can analyze the distance or differences between the identified subpopulations and thereby determine the particular biological events that occur within each subpopulation and map these events/distinctive features to the original biological data to permit biological interpretation and visualization.

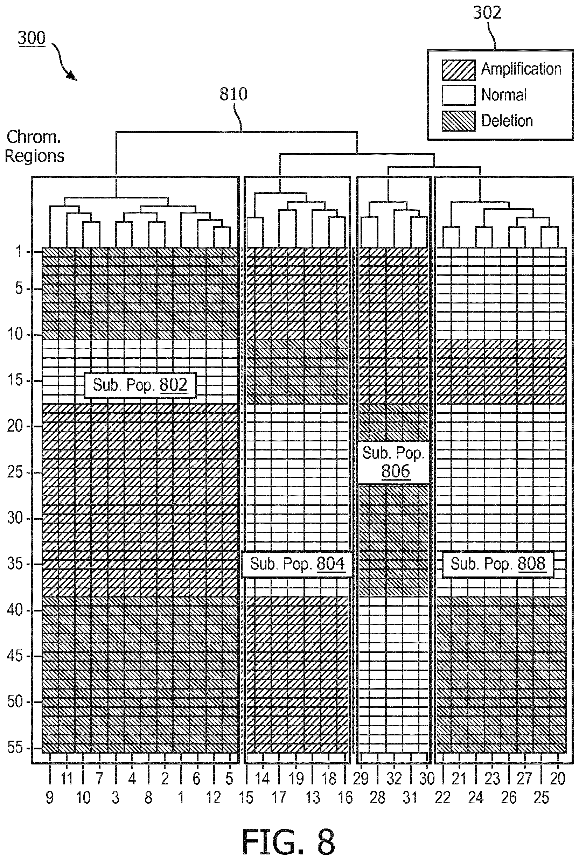

Referring again to FIGS. 1 and 2, at step 212, the subpopulation detection module 118 can output or direct the display of the identified subpopulations. For example, the subpopulation detection module 118 can direct the display of the identified subpopulations on the display device 104. Here, the identified subpopulations can be a simple listing of the identified subpopulations of the constituents. Alternatively, the output of the identified subpopulations can further include statistical characteristics. For example, FIG. 8 illustrates an example of a diagram 800 indicating the identified subpopulations for display at step 212. The display or diagram can include descriptive characteristics of inter-subpopulation similarities and/or inter-population dissimilarities. For example, FIG. 8 illustrates a cluster tree 810 that can be obtained by applying step 610. In addition, FIG. 8 also illustrates four subpopulations 802, 804, 806 and 808 of cohort members identified at step 210. In particular, FIG. 8 illustrates subpopulations of the cohort of FIG. 3. Similar to FIG. 3, the cohort members are identified on the horizontal axis of the diagram. As illustrated in FIG. 8, diagram 800 identifies the characteristics of each the subpopulations, indicating amplification, deletion or normal features at particular regions of the genomes of the subpopulations. In this example, the identified subpopulations are clonal populations. While only three characteristics are shown, it should be noted that more distinct characteristics can be displayed including, for example, partial deletions, complete deletions, and degrees of amplification. Although only copy number data is shown here for ease of illustration, it should be understood that characteristics of other categories of biological data, including, for example, mutations, gene or noncoding RNA expression data, DNA methylation data, histone modifications, DNA binding data (e.g. ChIPseq), RNA binding data, other types of genomic data, protein expression data, phosphoryltation data, ubiquitination data, acetylation data, and/or other types of proteomic data, in addition to other types of biological data described above, can be analyzed in accordance with method 200 and displayed at step 212. The data can be combined into one diagram or can be separated into data category-specific diagrams that each show characteristics from one category. Furthermore, the descriptive characteristics of inter-subpopulation similarities and/or inter-population dissimilarities can include values of the dissimilarity index, for example, values or differences between parent-child pairs delineating the subpopulations, to convey the degree to which subpopulations differ with respect to one or more categories of biological data. As noted above, high dissimilarity indices between the subpopulations can indicate high heterogeneity with multiple clonal and sub-clonal populations.

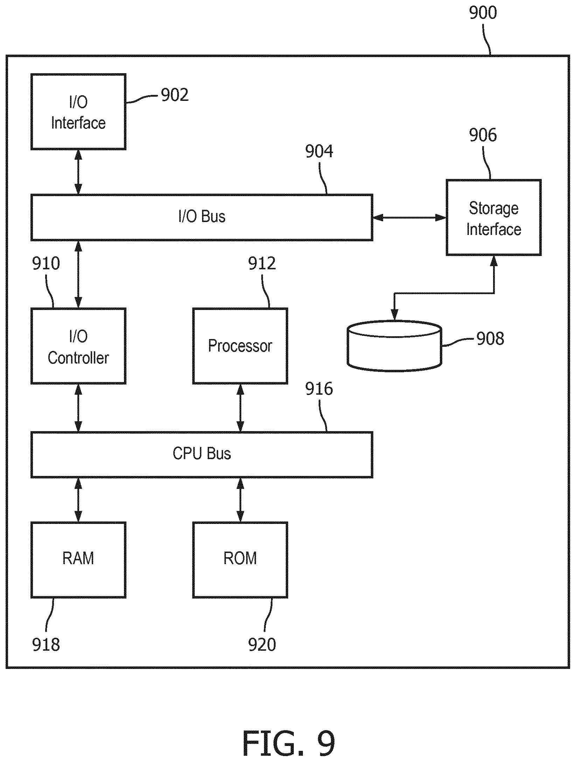

Referring now to FIG. 9, an exemplary computing system 900 by which method embodiments of the present principles described above can be implemented, is illustrated. The computing system 900 includes a hardware processor or controller 912 and a storage medium 908. The processor 912 can access random access memory (RAM) 918 and read only memory (ROM) 920 through a central processing unit (CPU) bus 916. In addition, the processor 912 can also access the computer-readable storage medium 908 through an input/output controller 910, an input/output bus 904 and a storage interface 906, as illustrated in FIG. 9. The system 900 can also include an input/output interface 902, which can be the user-interface 102 and can be coupled to a display device 104, keyboard, mouse, touch screen, external drives or storage mediums, etc., for the input and output of data to and from the system 900. In accordance with one exemplary embodiment, the processor 912 can access software instructions stored in the storage medium 908 and can access memories 918 and 920 to run the software instructions stored on the storage medium 908. In particular, the software instructions can implement or be the steps of methods 200, 400, 500 and/or 600. Alternatively, the software instructions that implement methods 200, 400, 500 and/or 600 can be encoded in a computer-readable signal medium, such as a radio frequency signal, an electrical signal or an optical signal.

As discussed above, the bioinformatics methods and systems described herein provide an efficient and accurate means for identifying subpopulations by transforming genomic data into discrete-time real-valued vector signals and applying a suitable frequency domain analysis. The embodiments described herein can be employed in any appropriate field utilizing bioinformatics technology. For example, as noted above, embodiments can be employed in clinical applications for purposes of detecting patterns of clonal evolution and tumor heterogeneity to determine aggressiveness of the tumor. In addition, as noted above, embodiments can be used in discovering new population outgrowth in bacterial infections, as well as in other applications. Further, the embodiments can be utilized in therapy design. For example, as noted above, the identification of subpopulations and dissimilarity indices can enable health care professionals to tailor drugs to each subpopulation, thereby significantly enhancing the chances of success of the treatment.

While several embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only. The invention is defined by the appended claims.

All definitions, as defined and used herein, should be understood to control over dictionary definitions, definitions in documents and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

## Claims

1. A system (100) for detecting subpopulations of constituents of at least one biological organism, said system being implemented by at least one hardware processor and comprising:

   a vector signal formulation module (112) configured to formulate biological data including genomic data compiled from a cohort of the constituents of said at least one biological organism as a set of discrete-time real valued vector signals within at least one data structure of a storage medium (108), wherein said formulating comprises defining the vector signals by characteristics of a genome and denoting regions of the genome as time values;
   a frequency domain analyzer (114) configured to perform frequency domain analysis on said vector signals of said biological data to compile spectral properties of said vector signals and to associate said spectral properties with said constituents of said cohort; and
   a subpopulation detection module (118) configured to identify said subpopulations of said at least one biological organism by applying a similarity metric to said spectral properties and to direct the display of a representation of said identified subpopulations; wherein said identifying comprises constructing (604) a population tree composed of parent-child pairs of said constituents and forming (606) a population dissimilarity index denoting dissimilarities between a parent and a child of each of said pairs based on said similarity metric.

2. The system of claim 1, wherein said performing comprises formulating at least a portion of said genomic data as at least one linear combination of distinct genomic events, preferably wherein said genomic events include at least one of copy number alteration events, mutations, gene expression data events or methylation data events.

3. The system of claim 1, wherein said identifying further comprises determining a total number of the subpopulations by detecting a total number of distinct peaks of said population dissimilarity index.

4. The system of claim 3, wherein said formulating comprises performing a principal component analysis on said genomic data to obtain principal components denoting linear combinations of genome regions and wherein said vector signals are composed of said principal components.

5. The system of claim 4, wherein said identifying comprises performing a clustering procedure on a combination of said vector signals and said spectral properties by employing said total number of distinct peaks as a height cutoff for said procedure corresponding to the total number of the subpopulations.

6. A method (200) for detecting subpopulations of constituents of at least one biological organism, said method being implemented by at least one hardware processor and comprising:

   formulating (204) biological data including genomic data compiled from a cohort of the constituents of said at least one biological organism as a set of discrete-time real valued vector signals within at least one data structure of a storage medium; wherein said formulating comprises defining the vector signals by characteristics of a genome and denoting regions of the genome as time values;

performing (206) frequency domain analysis on said vector signals of said biological data to compile spectral properties of said vector signals;

associating (208) said spectral properties with said constituents of said cohort; and

identifying (210) said subpopulations of said constituents of the at least one biological organism by applying a similarity metric to said spectral properties; wherein said identifying comprises constructing (604) a population tree composed of parent-child pairs of said constituents and forming (606) a population dissimilarity index denoting dissimilarities between a parent and a child of each of said pairs based on said similarity metric.

7. The method of claim 6, wherein said spectral properties include at least one of power spectral density or total spectral energy.

8. The method of claim 6, wherein said performing comprises formulating at least a portion of said genomic data as at least one linear combination of distinct genomic events.

9. The method of claim 8, wherein said genomic events include at least one of copy number alteration events, mutations, gene expression data events or methylation data events.

10. The method of claim 6, wherein said performing comprises determining (508) at least one of power spectral density or total spectral energy for each genomic event of said distinct genomic events.

11. The method of claim 6, wherein said identifying further comprises determining (608) a total number of the subpopulations by detecting a total number of distinct peaks of said population dissimilarity index.

12. The method of claim 11, wherein said formulating comprises performing (408) a principal component analysis on said genomic data to obtain principal components denoting linear combinations of genome regions and wherein said vector signals are composed of said principal components.

13. The method of claim 12, wherein said identifying comprises performing (612) a clustering procedure on a combination of said vector signals and said spectral properties by employing said total number of distinct peaks as a height cutoff for said procedure corresponding to the total number of the subpopulations.

14. The method of claim 6, further comprising:
displaying (212) a representation of said identified subpopulations.

15. A computer-readable medium (908) comprising a computer-readable program that, when executed on a computer, enables the computer to perform the method of any one of claims 6 to 14.

**Patentansprüche**

1. System (100) zum Nachweisen von Subpopulationen von Bestandteilen mindestens eines biologischen Organismus, wobei das System durch mindestens einen Hardwareprozessor implementiert ist und Folgendes umfasst:

ein Vektorsignal-Formulierungsmodul (112), das dazu konfiguriert ist, biologische Daten einschließlich genomischer Daten, die aus einer Kohorte der Bestandteile des mindestens einen biologischen Organismus zusammengestellt wurden, als einen Satz von diskreten reellwertigen Vektorsignalen innerhalb mindestens einer Datenstruktur eines Speichermediums (108) zu formulieren, wobei das Formulieren das Definieren der Vektorsignale durch Merkmale eines Genoms und das Bezeichnen von Regionen des Genoms als Zeitwerte umfasst;

einen Frequenzraum-Analysator (114), der dazu konfiguriert ist, eine Frequenzraum-Analyse an den Vektorsignalen der biologischen Daten durchzuführen, um spektrale Eigenschaften der Vektorsignale zusammenzustellen und die spektralen Eigenschaften den Bestandteilen der Kohorte zuzuordnen; und

ein Subpopulationserfassungsmodul (118), das dazu konfiguriert ist, die Subpopulationen des mindestens einen biologischen Organismus durch Anwendung einer Ähnlichkeitsmetrik auf die spektralen Eigenschaften zu identifizieren und die Anzeige einer Darstellung der identifizierten Subpopulationen zu steuern; wobei das Identifizieren das Konstruieren (604) eines Populationsbaums umfasst, der aus Paaren von übergeordneten und untergeordneten Elementen der Bestandteile zusammengesetzt ist sowie das Bilden (606) eines Populations-Unähnlichkeitsindexes, der Unähnlichkeiten zwischen einem übergeordneten und einem untergeordneten Element jedes der Paare auf der Grundlage der Ähnlichkeitsmetrik angibt.

2. System nach Anspruch 1, wobei das Durchführen das Formulieren von mindestens einem Teil der genomischen Daten als mindestens eine lineare Kombination von unterschiedlichen genomischen Ereignissen umfasst, wobei die genomischen Ereignissen vorzugsweise mindestens eines der folgenden Ereignisse umfassen: Kopienzahländerungs-Ereignisse, Mutationen Genexpressionsdaten Ereignisse oder Methylierungsdaten-Ereignisse.

3. System nach Anspruch 1, wobei das Identifizieren ferner das Bestimmen einer Gesamtzahl der Subpopulationen durch Erfassen einer Gesamtzahl unterschiedlicher Spitzenwerte des Populations-Unterschiedsindex umfasst.

4. System nach Anspruch 3, wobei das Formulieren das Durchführen einer Hauptkomponenten-Analyse an den genomischen Daten umfasst, um Hauptkomponenten zu erhalten, die lineare Kombinationen von Genomregionen kennzeichnen, und wobei die Vektorsignale aus den Hauptkomponenten zusammengesetzt sind.

5. System nach Anspruch 4, wobei das Identifizieren das Durchführen einer Gruppierungsprozedur an einer Kombination der Vektorsignale und der spektralen Eigenschaften umfasst, indem die Gesamtzahl der unterschiedlichen Spitzenwerte als Höhenbegrenzung für die Prozedur entsprechend der Gesamtzahl der Subpopulationen verwendet wird.

6. Verfahren (200) zum Nachweisen von Subpopulationen von Bestandteilen mindestens eines biologischen Organismus, wobei das Verfahren durch mindestens einen Hardwareprozessor implementiert ist und Folgendes umfasst:

Formulieren (204) biologischer Daten, einschließlich genomischer Daten, die aus einer Kohorte der Bestandteile des mindestens einen biologischen Organismus zusammengestellt wurden, als Satz von zeitdiskreten reellwertigen Vektorsignalen in mindestens einer Datenstruktur eines Speichermediums, wobei das Formulieren das Definieren der Vektorsignale durch Eigenschaften eines Genoms und das Bezeichnen von Regionen des Genoms als Zeitwerte umfasst;
Durchführen (206) einer Frequenzraum-Analyse an den Vektorsignalen der biologischen Daten, um spektrale Eigenschaften der Vektorsignale zusammenzustellen;
Zuordnen (208) der spektralen Eigenschaften mit den Bestandteilen der Kohorte; und
Identifizieren (210) der Subpopulationen der Bestandteile des mindestens einen biologischen Organismus durch Anwenden einer Ähnlichkeitsmetrik auf die spektralen Eigenschaften, wobei das Identifizieren das Konstruieren (604) eines Populationsbaums umfasst, der aus Paaren von übergeordneten und untergeordneten Elementen der Bestandteile zusammengesetzt ist sowie das Bilden (606) eines Populations-Unähnlichkeitsindexes, der Unähnlichkeiten zwischen einem übergeordneten und einem untergeordneten Element jedes der Paare auf der Grundlage der Ähnlichkeitsmetrik angibt.

7. Verfahren nach Anspruch 6, wobei die spektralen Eigenschaften die spektrale Leistungsdichte und/oder die gesamte spektrale Energie umfassen.

8. Verfahren nach Anspruch 6, wobei das Durchführen das Formulieren mindestens eines Teils der genomischen Daten als mindestens eine lineare Kombination verschiedener genomischer Ereignisse umfasst.

9. Verfahren nach Anspruch 8, wobei die genomischen Ereignissen vorzugsweise mindestens eines der folgenden Ereignisse umfassen: Kopienzahländerungs-Ereignisse, Mutationen Genexpressionsdaten Ereignisse oder Methylierungsdaten-Ereignisse.

10. Verfahren nach Anspruch 6, wobei das Durchführen das Bestimmen (508) von spektraler Leistungsdichte und/oder gesamter spektraler Energie für jedes genomische Ereignis der verschiedenen genomischen Ereignisse umfasst.

11. Verfahren nach Anspruch 6, wobei das Identifizieren ferner das Bestimmen (608) einer Gesamtzahl der Subpopulationen durch Erfassen einer Gesamtzahl von unterschiedlichen Spitzenwerten des Populations-Unähnlichkeitsindexes umfasst.

12. Verfahren nach Anspruch 11, wobei das Formulieren das Durchführen (408) einer Hauptkomponentenanalyse an den genomischen Daten umfasst, um Hauptkomponenten zu erhalten, die lineare Kombinationen von Genomregionen kennzeichnen, und wobei die Vektorsignale aus den Hauptkomponenten zusammengesetzt sind.

13. Verfahren nach Anspruch 12, wobei das Identifizieren das Durchführen (612) einer Gruppierungsprozedur an einer Kombination der Vektorsignale und der spektralen Eigenschaften umfasst, indem die Gesamtzahl der unterschied-

**EP 3 304 384 B1**

lichen Spitzenwerte als Höhenbegrenzung für die Prozedur entsprechend der Gesamtzahl der Subpopulationen verwendet wird.

**14.** Verfahren nach Anspruch 6, das ferner Folgendes umfasst:
Anzeigen (212) einer Darstellung der identifizierten Subpopulationen.

**15.** Computerlesbares Medium (908) mit einem computerlesbaren Programm, das, wenn es auf einem Computer ausgeführt wird, es dem Computer ermöglicht, das Verfahren nach einem der Ansprüche 6 bis 14 auszuführen.

**Revendications**

**1.** Système (100) de détection de sous-populations de constituants d'au moins un organisme biologique, ledit système étant mis en œuvre par au moins un processeur matériel et comprenant :

un module de formulation de signal vectoriel (112) configuré pour formuler des données biologiques comprenant des données génomiques compilées à partir d'une cohorte de constituants d'au moins un organisme biologique en tant qu'ensemble de signaux vectoriels de valeur réelle en temps discret à l'intérieur d'au moins une structure de données d'un moyen de stockage (108), dans lequel ladite formulation comprend la définition des signaux vectoriels par les caractéristiques d'un génome et désignant des régions du génome en tant que valeurs temporelles ;
un analyseur de domaine de fréquence (114) configuré pour réaliser une analyse de domaine de fréquence sur lesdits signaux vectoriels desdites données biologiques afin de compiler les propriétés spectrales desdits signaux vectoriels et d'associer lesdites propriétés spectrales auxdits constituants de ladite cohorte ; et
un module de détection de sous-population (118) configuré pour identifier lesdites sous-populations d'au moins un organisme biologique par application d'une métrique similaire auxdites propriétés spectrales et pour diriger l'affichage d'une représentation desdites sous-populations identifiées ; dans lequel ladite identification comprend la construction (604) d'une arborescence de population composée de paires de parent-enfant desdits constituants et formant (606) un indice de dissimilarité de population désignant des dissimilarités entre un parent et un enfant de chacune desdites paires en se basant sur ladite métrique de similarité.

**2.** Système selon la revendication 1, dans lequel ladite réalisation comprend la formulation d'au moins une partie desdites données génomiques en tant qu'au moins une combinaison linéaire des événements génomiques distincts, de préférence dans lequel lesdits événements génomiques comprennent au moins l'un des événements d'altération d'un nombre de copies, mutations, événements de données d'expression de gènes ou événements de données de méthylation.

**3.** Système selon la revendication 1, dans lequel ladite identification comprend en outre la détermination d'un nombre total de sous-populations par la détection d'un nombre total de pics distincts dudit indice de dissimilarité de population.

**4.** Système selon la revendication 3, dans lequel ladite formulation comprend la réalisation d'une analyse de composant principal sur lesdites données génomiques afin d'obtenir des composants principaux désignant des combinaisons linéaires des régions de génome et dans lequel lesdits signaux vectoriels sont composés desdits composants principaux.

**5.** Système selon la revendication 4, dans lequel ladite identification comprend la réalisation d'une procédure de regroupement sur une combinaison desdits signaux vectoriels et desdites propriétés spectrales par utilisation dudit nombre total de pics distincts en tant que hauteur seuil pour ladite procédure correspondant au nombre total de sous-populations.

**6.** Procédé (200) de détection des sous-populations de constituants d'au moins un organisme biologique, ledit procédé étant mis en œuvre par au moins un processeur matériel et comprenant :

la formulation (204) des données biologiques comprenant des données génomiques compilées à partir d'une cohorte de constituants d'au moins un organisme biologique en tant qu'ensemble de signaux vectoriels de valeur réelle en temps discret à l'intérieur d'au moins une structure de données d'un moyen de stockage ; dans lequel ladite formulation comprend la définition des signaux vectoriels par caractéristiques d'un génome et la désignation de régions de génome en tant que valeurs temporelles ;

17

la réalisation (206) d'analyse de domaine de fréquence sur lesdits signaux vectoriels desdites données biologiques afin de compiler les propriétés spectrales desdits signaux vectoriels ;

l'association (208) desdites propriétés spectrales avec lesdits constituants de ladite cohorte ; et

l'identification (210) desdites sous-populations desdits constituants d'au moins un organisme biologique par application d'une métrique de similarité auxdites propriétés spectrales ; dans lequel ladite identification comprend la construction (604) d'une arborescence de population composée de paires de parent-enfant desdits constituants et la formation (606) d'un indice de dissimilarité de population désignant les dissimilarités entre un parent et un enfant de chacune desdits paires sur la base de ladite métrique de similarité.

7. Procédé selon la revendication 6, dans lequel lesdites propriétés spectrales comprennent au moins une densité spectrale de puissance ou une énergie spectrale totale.

8. Procédé selon la revendication 6, dans lequel ladite réalisation comprend la formulation d'au moins une partie des données génomiques en tant qu'au moins une combinaison linéaire d'événements génomiques distincts.

9. Procédé selon la revendication 8, dans lequel lesdits événements génomiques comprennent au moins l'un des événements d'altération d'un nombre de copies, mutations, événements de données d'expression de gènes ou événements de données de méthylation.

10. Procédé selon la revendication 6, dans lequel ladite réalisation comprend la détermination (508) d'au moins une densité spectrale de puissance ou d'énergie spectrale totale pour chaque événement génomique desdits événements génomiques distincts.

11. Procédé selon la revendication 6, dans lequel l'identification comprend en outre la détermination (608) d'un nombre total de sous-populations par détection d'un nombre total de pics distincts dudit indice de dissimilarité de population.

12. Procédé selon la revendication 11, dans lequel ladite formulation comprend la réalisation (408) d'une analyse de composant principal sur lesdites données génomiques afin d'obtenir des composants principaux désignant des combinaisons linéaires de régions de génome et dans lequel lesdits signaux vectoriels sont composés desdits composants principaux.

13. Procédé selon la revendication 12, dans lequel ladite identification comprend la réalisation (612) d'une procédure de regroupement sur une combinaison desdits signaux vectoriels et desdites propriétés spectrales par l'utilisation dudit nombre total de pics distincts en tant que hauteur seuil pour ladite procédure correspondant au nombre total des sous-populations.

14. Procédé selon la revendication 6, comprenant en outre :
l'affichage (212) d'une représentation desdites sous-populations identifiées.

15. Support lisible par ordinateur (908) comprenant un programme lisible par ordinateur qui, exécuté sur un ordinateur, permet à l'ordinateur de réaliser le procédé de l'une quelconque des revendications 6 à 14.

100

UI ─ 102

Dsply Dev. ─ 104

112

114

118

VS Form. Mod.

FD Anlzr

Subpop. Det. Mod.

Cntrlr ─ 110

108

─ 106

# FIG. 1

EP 3 304 384 B1

FIG. 2

FIG. 3

400

- - - - - - - - - - - - - - - - - - -
Identify and separate outliers — 402
- - - - - - - - - - - - - - - - - - -

↓

Formulate biological data as discrete-time
real valued vector signals — 404

↓

- - - - - - - - - - - - - - - - - - -
Split data into sets of adjacent regions — 406
- - - - - - - - - - - - - - - - - - -

↓

- - - - - - - - - - - - - - - - - - -
Perform principal component analysis on
each region and identify principal components — 408
- - - - - - - - - - - - - - - - - - -

↓

- - - - - - - - - - - - - - - - - - -
Formulate principal components as feature
vector signals — 410
- - - - - - - - - - - - - - - - - - -

# FIG. 4

500

Transform each vector signal into the
frequency domain — 502

↓

504

Determine spectral properties of each vector signal

506

508

- - - - - - - - - - - - - - - -
Extract biological events
- - - - - - - - - - - - - - - -
→
- - - - - - - - - - - - - - - -
For each biological event,
determine PSD and/or TSE
- - - - - - - - - - - - - - - -

↓

- - - - - - - - - - - - - - - -
Split frequency range
- - - - - - - - - - - - - - - -
→
- - - - - - - - - - - - - - - -
Determine APSD and ATSE
- - - - - - - - - - - - - - - -

510

512

# FIG. 5

600

602 — Construct/Identify root for population tree

604 — Construct population tree composed of parent-child pairs

606 — Form population dissimilarity index based on similarity metric

608 — Determine total number of subpopulations by analyzing dissimilarity index

610 — Identify subpopulations of cohort

612 — Perform clustering procedure using determined total number of subpopulations as a height cutoff

614 — Identify distinctive features by analyzing inter-subpopulation distance components

# FIG. 6

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003097227 A
- US 2009129647 A

- WO 0173602 A

**Non-patent literature cited in the description**

- **MERTINS et al.** Integrated proteomic analysis of post-translational modifications by serial enrichment. *Nature Methods,* 2013, vol. 10, 634-637